Europäisches Patentamt

European Patent Office

Office européen des brevets

(11) Publication number: **0 105 657 B1**

## EUROPEAN PATENT SPECIFICATION

(45) Date of publication of patent specification: **27.05.92**   (51) Int. Cl.5: **A61K 7/48**

(21) Application number: **83305511.4**

(22) Date of filing: **20.09.83**

(54) **Cosmetic compositions containing glycerine.**

(30) Priority: **30.09.82 US 432070**
**06.06.83 US 501626**

(43) Date of publication of application:
**18.04.84 Bulletin 84/16**

(45) Publication of the grant of the patent:
**27.05.92 Bulletin 92/22**

(84) Designated Contracting States:
**BE DE FR GB IT NL SE**

(56) References cited:
**DE-A- 855 615**
**DE-A- 2 601 049**
**GB-A- 1 575 201**
**GB-A- 2 002 652**
**US-A- 3 932 614**

(73) Proprietor: **Lancaster Group AG**
**Mainzerstrasse 15**
**W-6200 Wiesbaden(DE)**

(72) Inventor: **Brechner, Stanley**
**28 Carteret Street**
**West Orange, NJ 07052(US)**
Inventor: **Lorenz, Jill**
**745 Union Avenue**
**Lyndhurst, NJ 07071(US)**

(74) Representative: **Jung, Elisabeth, Dr. et al**
**Dr. Elisabeth Jung Dr. Jürgen Schirdewahn**
**Dipl.-Ing. Claus Gernhardt Patentanwälte**
**Clemensstrasse 30 Postfach 40 14 68**
**W-8000 München 40(DE)**

EP 0 105 657 B1

Rank Xerox (UK) Business Services

**Description**

The present invention relates to cosmetic compositions, containing glycerin, which are useful for imparting moisture to the skin of humans.

Hitherto, glycerin has been suggested for use in the treatment of dry skin conditions but one disadvantage of any composition which contains a high concentration of glycerin is that it leaves a wet and coated feeling on the skin which is cosmeticallyunacceptable to the user.

U.K. Patent Application GB-A-1,575,201 which is equivalent to DE-A-2,601,049 and US-A-3,932,614 discloses a cosmetic preparation comprising water, carboxypolymethylene and 1 to 50% by weight of a lubricating agent selected from the group consisting of chlorotrifluoroethylene resin, the homopolymer of tetrafluoroethylene, the homopolymer of hexafluoropropylene, and the copolymer of tetrafluoroethylene and hexafluoropropylene having the formula $-(CF_2CF_2-CF_2CFCF_3)n$ where n is an integer corresponding to the number of repeating units in the copolymerized chain.

It has now been found that the above disadvantage can be overcome or reduced by including polymer particles in a cosmetic composition containing high levels of glycerin. At the same time, the skin moisturising properties of glycerin can be efficiently utilised.

According to the present invention, there is provided a cosmetic composition for moisturising skin, comprising from 10% to 30%, by weight of the composition, of glycerin, from 0.2% to 5%, by weight of the composition, of particulate polymer or copolymer which is capable of reducing the lubricity of glycerin, and a cosmetically acceptable carrier.

Preferably, the amount of glycerin is from 15% to 30%. Other suitable amounts of glycerin are from 10% to 20%, or 20% to 30%. The preferred amount of polymer or copolymer is from 0.2% to 2%, particularly preferably about 0.5%.

The polymer or copolymer in the composition of the invention should be capable of reducing the lubricity of the glycerin so that the composition has a non-oily, non-wet feel on the users skin.

Polymers which are particularly useful in the composition according to the invention are acrylic copolymers such as those described in United States Patent Nos. 4,043,952 and 4,090,013, and starch-graft copolymers such as those described in US Patent Nos. 3,935,099, 3,981,100, 3,985,616, 4,069,177, 4,045,387, 4,116,899, 4,134,863 and 4,194,998.

The composition of the invention preferably contains from about 0.5% to about 1% of acrylic or starch-graft copolymer, particularly preferably about 0.5%.

Other useful polymers are polyethylenemicrospheres, such as Polymist B-6[R] (Allied Chemical), and polyamide powder, such as Orgasol 2002D[R] (Autochem).

The polymer or copolymer is in the form of particles having an average particle size in the range of 5 to 15$\mu$m (microns), preferably 5 to 7$\mu$m (microns).

In a further aspect, the present invention provides a method of imparting moisture to skin which comprises applying to skin an effective amount of a composition of the invention.

The composition of the invention may include any of the ingredients customarily used in cosmetic formulations intended for application to skin. Typical ingredients are preservatives, perfumes, colouring agents and anti oxidants. The cosmetically acceptable carrier may comprise conventional materials such as oil-in-water or water-in-oil emulsions.

The composition of the invention may be prepared by admixture. Accordingly, the invention also provides a process for preparing the cosmetic composition of the invention, which comprises admixing the components of the composition in the required proportions.

The admixing should be carried out so that the polymer or copolymer is uniformly dispersed throughout the final composition.

The oil-in-water or water-in-oil emulsion may be prepared according to general methods described in UK Patent Specification Nos. 1 489 133 and 2 026 319. The emulsion may then be mixed with the glycerine and copolymer or polymer to produce the final composition of the invention.

The invention is now illustrated by means of the following examples.

EXAMPLE 1

The following moisturizing composition was prepared:

| Glycerin | 30% by weight |  |
|---|---|---|
| Permasorb 10 | 0.50 | " |
| Cetyl alcohol | 1.00 | " |
| Polyethylene Glycol 2 laurate | 2.00 | " |
| Dimethyl silicone | 0.50 | " |
| Stearic acid | 3.00 | " |
| Potassium hydroxide | 0.30 | " |
| Finsolv TN | 4.00 | " |
| Preservative | q.s. |  |
| Fragrance | q.s. |  |
| Color | q.s. |  |
| Water to make | 100.00% | " |

Permasorb 10 - Acrylic copolymer (National Starch and Chemical Co.)
Finsolv TN - C 12-15 alcohols Benzoate (Grain Processing Corp.)

EXAMPLE 2

The following moisturizing composition was prepared:

| Glycerin | 30% by weight |  |
|---|---|---|
| Permasorb 10 | 0.50 | " |
| Cetyl alcohol | 1.00 | " |
| Polyethylene Glycol 2 laurate | 2.00 | " |
| Dimethyl silicone | 0.50 | " |
| Stearic acid | 3.00 | " |
| Potassium hydroxide | 0.30 | " |
| Preservative | q.s. |  |
| Fragrance | q.s. |  |
| Color | q.s. |  |
| Water to make | 100.00% | " |

Permasorb 10 - Acrylic copolymer

EXAMPLE 3

The following moisturizing composition was prepared

3

| Glycerin | 20% by weight |
| Permasorb 10 | 0.50 " |
| Cetyl alcohol | 1.00 " |
| Polyethylene Glycol 2 laurate | 2.00 " |
| Dimethyl silicone | 0.50 " |
| Stearic acid | 3.00 " |
| Potassium hydroxide | 0.30 " |
| Finsolv TN | 4.00 " |
| Preservative | q.s. |
| Fragrance | q.s. |
| Color | q.s. |
| Water to make | 100.00% " |

Permasorb 10 - Acrylic copolymer
Finsolv TN - C 12-15 alcohols Benzoate

EXAMPLE 4

The following moisturizing composition was prepared:

| Glycerin | 30% by weight |
| Permasorb 10 | 0.2 " |
| Cetyl alcohol | 0.5 " |
| Polyethylene Glycol 2 laurate | 2.0 " |
| Dimethyl silicone | 0.2 " |
| Stearic Acid | 3.0 " |
| Potassium hydroxide | 0.2 " |
| Preservative | q.s. |
| Fragrance | q.s. |
| Water to make | 100.00% " |

Permasorb 10 - Acrylic copolymer

EXAMPLE 5

The following moisturizing composition was prepared:

4

| Glycerin | 15% by weight |
|---|---|
| Permasorb 10 | 2.0 " |
| Cetyl alcohol | 0.5 " |
| Polyethylene Glycol 2-laurate | 2.0 " |
| Dimethyl silicone | 0.2 " |
| Stearic Acid | 3.0 " |
| Potassium hydroxide | 0.2 " |
| Preservative | q.s. |
| Fragrance | q.s. |
| Water to make | 100% " |

Permasorb 10 - Acrylic copolymer

EXAMPLE 6

The following moisturizing composition was prepared:

| Glycerin | 30% by weight |
|---|---|
| Permasorb 10 | 0.2 " |
| Water Lock A-100 | 0.2 " |
| Cetyl alcohol | 1.0 " |
| Polyethylene Glycol 2-laurate | 2.0 " |
| Dimethyl silicone | 0.5 " |
| Stearic Acid | 3.0 " |
| Potassium hydroxide | 0.2 " |
| Preservative | q.s. |
| Fragrance | q.s. |
| Water to make | 100% " |

Permasorb 10 - Acrylic copolymer
Water Lock A-100 - Starch graft copolymer

EXAMPLE 7

The following moisturizing composition was prepared:

|  |  |  |
|---|---|---|
| Glycerin | 30% by weight | |
| Water Lock A-100 | 0.5 | " |
| Cetyl alcohol | 0.5 | " |
| Polyethylene Glycol 2-laurate | 2.0 | " |
| Dimethyl silicone | 0.2 | " |
| Stearic Acid | 3.0 | " |
| Potassium hydroxide | 0.2 | " |
| Preservative | q.s. | |
| Fragrance | q.s. | |
| Water to make | 100% | " |

Water Lock A-100 - Starch graft copolymer

## EXAMPLE 8

The following moisturizing composition was prepared:

|  |  |  |
|---|---|---|
| Sorbitan Stearate | 1.50 | % by weight |
| Polysorbate 60® (ICI Chemicals) | 2.00 | " |
| Glyceryl stearate & PEG 100 Stearate | 5.00 | " |
| Cetyl alcohol | 6.50 | " |
| Mineral Oil | 6.00 | " |
| Isopropyl myristate | 3.00 | " |
| Silicone 200 Fluid | 1.00 | " |
| Polymist B6® | 5.00 | " |
| Orgasol 2002D® | – | |
| Nipabutyl® (Nipa Chemicals) | 0.02 | " |
| Deionised water | 58.66 | " |
| Glycerine | 10.00 | " |
| Triethanolamine | 0.20 | " |
| Imidazolidinyl Urea | 0.30 | " |
| Carbomer 941® (good rich Ltd) | 0.10 | " |
| Perfume | 0.40 | " |
| Nipastat® (Nipa Chemicals) | 0.30 | " |
|  | 100.00 | |

## EXAMPLE 9

The following moisturizing composition was prepared:

| | | |
|---|---|---|
| Stearic acid | 1.80 | % by weight |
| Mineral Oil | 4.00 | " |
| Cetyl alcohol | 2.00 | " |
| Glyceryl stearate & PEG 100 Stearate | 2.00 | " |
| Silicone 200 Fluid | 0.50 | " |
| Nipabutyl® | 0.02 | " |
| Polymist B6® | 5.00 | " |
| Orgasol 2002D® | - | |
| Deionised water | 73.03 | " |
| Glycerine | 10.00 | " |
| Triethanolamine | 0.60 | " |
| Nipastat® | 0.30 | " |
| Imidazolidinyl Urea | 0.30 | " |
| Carbomer 941® | 0.05 | " |
| Perfume | 0.40 | " |
| | 100.00 | " |

EXAMPLE 10

| | | |
|---|---|---|
| Ceresin Wax | 2.00 | % by weight |
| Mineral oil | 20.00 | " |
| PEG 20/Dodecyl Glycol Copolymer | 1.00 | " |
| Elfacos C26® (Synthetic wax, Akzochemie) | 1.00 | " |
| Sorbitol (70%) | 9.00 | " |
| Nipabutyl® | 0.06 | " |
| Polymist B6® | 5.00 | " |
| Orgasol 2002D®. | - | |
| Deionised water | 51.22 | " |
| Glycerine | 10.00 | " |
| Nipastat® | 0.30 | " |
| 2-Bromo-2-Nitropropane-1,3-Diol | 0.02 | " |
| Perfume | 0.40 | " |
| | 100.00 | " |

The following moisturizing composition was prepared:

EXAMPLE 11

The following moisturizing composition was prepared:

7

| | | |
|---|---|---|
| Glyceryl oleate and propylene glycol | 2.00 | % by weight |
| Liquid paraffin | 17.00 | " |
| PEG 40/Dodecyl glycol Copolymer | 3.00 | " |
| Nipabutyl® | 0.06 | " |
| Petrolatum | 5.00 | " |
| Polymist B6® | 5.00 | " |
| Orgasol 2002D® | – | |
| Deionised water | 56.74 | " |
| Glycerine | 10.00 | " |
| Nipastat® | 0.30 | " |
| Imidazolidinyl Urea | 0.50 | " |
| Perfume | 0.40 | " |
| | 100.0 | " |

Similar compositions to those of Examples 8 to 11 were prepared in which the Polymist B-6 was replaced by the same percentage by weight of Orgasol 2002D.

## Claims

1. A cosmetic composition for moisturising skin, comprising from 10% to 30%, by weight of the composition, of glycerin, from 0.2% to 5%, by weight of the composition, of a particulate polymer or copolymer which is capable of reducing the lubricity of glycerin, and a cosmetically acceptable carrier.

2. A composition according to claim 1, comprising from 15% to 30%, by weight of glycerin.

3. A composition according to claim 1 or claim 2, comprising from 0.2% to 2%, by weight of polymer or copolymer.

4. A composition according to any one of claims 1 to 3, in which the copolymer comprises an acrylic copolymer or starch graft copolymer.

5. A composition according to claim 4, in which the copolymer is present in an amount of from 0.5% to 1% by weight.

6. A composition according to any one of claims 1 to 3, in which the polymer or copolymer comprises polyethylene microspheres or polyamide powder.

7. A process for preparing a cosmetic composition according to any one of claims 1 to 6, which comprises admixing the components of the composition in the required proportions.

## Revendications

1. Composition cosmétique pour hydrater la peau, comprenant de 10% à 30% en poids de la composition de glycérine, de 0,2% à 5% de poids de la composition d'un polymère ou d'un copolymère particulaire qui est capable de réduire l'onctuosité de la glycérine et un support acceptable du point de vue cosmétique.

2. Composition suivant la revendication 1, comprenant de 15% à 30% en poids de glycérine.

3. Composition suivant la revendication 1 ou la revendication 2, comprenant de 0,2% à 2% en poids de polymère ou de copolymère.

4. Composition suivant l'une quelconque des revendications 1 à 3, dans laquelle le copolymère comprend un copolymère acrylique ou un copolymère à greffon d'amidon.

**5.** Composition suivant la revendication 4, dans laquelle le copolymère est présent en une quantité de 0,5% à 1% en poids.

**6.** Composition suivant l'une quelconque des revendications 1 à 3, dans laquelle le polymère ou le copolymère comprend des microsphères de polyéthylène ou une poudre de polyamide.

**7.** Procédé pour préparer une composition cosmétique suivant l'une quelconque des revendications 1 à 6, qui comprend le mélange des composants de la composition selon les proportions désirées.

**Patentansprüche**

**1.** Eine kosmetische Zusammensetzung, die dazu dient, der Haut Feuchtigkeit zu verleihen, umfassend 10 bis 30 % Glycerin, bezogen auf das Gewicht der Zusammensetzung, 0,2 bis 5% eines teilchenförmigen Polymers oder Copolymers, bezogen auf das Gewicht der Zusammensetzung, welches dazu in der Lage ist, die Schlüpfrigkeit von Glycerin zu reduzieren, und einen kosmetisch verträglichen Träger.

**2.** Eine Zusammensetzung nach Anspruch 1, umfassend 15 bis 30 Gewichtsprozent Glycerin.

**3.** Eine Zusammensetzung nach Anspruch 1 oder 2, umfassend 0,2 bis 2 Gewichtsprozent eines Polymers oder Copolymers.

**4.** Eine Zusammensetzung nach einem der Ansprüche 1 bis 3, in welcher das Copolymer ein Acryl-Copolymer oder ein Stärke-Pfropfmischpolymerisat ist.

**5.** Eine Zusammensetzung nach Anspruch 4, in welcher das Copolymer in einer Menge von 0,5 bis 1 Gewichtsprozent vorliegt.

**6.** Eine Zusammensetzung nach einem der Ansprüche 1 bis 3, in welcher das Polymer oder Copolymer in Form von Polyäthylen-Mikrokügelchen oder Polyamidpulver vorliegt.

**7.** Ein Verfahren zur Herstellung einer kosmetischen Zusammensetzung nach einem der Ansprüche 1 bis 6, welches das Zumischen der Komponenten der Zusammensetzung in den erforderlichen Anteilsmengen umfaßt.